# FASCICULE DE BREVET EUROPEEN

(11) **EP 1 633 800 B1**
(45) Date de publication et mention de la délivrance du brevet: **10.08.2011**
(21) Numéro de dépôt: 04742877.6
(22) Date de dépôt: 28.05.2004
(51) Int. Cl.: C08G 69/10, A61K 47/34, A61K 47/42, A61K 9/51, C08G 69/48

(54) **POLYAMINOACIDES FONCTIONNALISES PAR AU MOINS UN GROUPEMENT HYDROPHOBE ET LEURS APPLICATIONS NOTAMMENT THERAPEUTIQUES**
MIT MINDESTENS EINER HYDROPHOBEN GRUPPE FUNKTIONALISIERTE POLYAMINOSÄUREN UND ANWENDUNGEN DAVON, INSBESONDERE THERAPEUTISCHE ANWENDUNGEN
POLYAMINO ACIDS FUNCTIONALISED WITH AT LEAST ONE HYDROPHOBIC GROUP AND APPLICATIONS THEREOF PARTICULARLY THERAPEUTIC APPLICATIONS

(30) Priorité: 28.05.2003 FR 0350190
(43) Date de publication de la demande: 15.03.2006
(73) Titulaire: FLAMEL TECHNOLOGIES, 69200 Vénissieux (FR)
(72) Inventeur: ANGOT, Stéphanie, 33200 Bordeaux (FR); BREYNE, Olivier, F-69004 LYON (FR); CHAN, You-Ping, F-69008 LYON (FR)
(74) Mandataire: Fleurance, Raphaël
(86) Numéro de dépôt international: PCT/FR2004/050209
(87) Numéro de publication internationale: WO 2004/108796

(56) Documents cités:
- EP-A- 0 179 023
- WO-A-00/30618
- WO-A-00/78791
- WO-A-87/03891
- WO-A-96/29991
- US-A1- 2002 169 125

## Description

La présente invention concerne des nouveaux matériaux à base de polyaminoacides biodégradables, utiles notamment pour la vectorisation de principe(s) actif(s) (PA).

L'invention vise aussi de nouvelles compositions pharmaceutiques, cosmétiques, diététiques ou phytosanitaires à base de ces polyaminoacides. Ces compositions peuvent être du type de celles permettant la vectorisation de PA et se présentant de préférence sous forme d'émulsions, de micelles, de particules, de gels, d'implants ou de films.

Les PA considérés sont, avantageusement, des composés biologiquement actifs et qui peuvent être administrés à un organisme animal ou humain par voie orale, parentérale, nasale, vaginale, oculaire, sous-cutanée, intraveineuse, intra-musculaire, intradermique, intrapéritonéale, intracérébrale, buccale, etc.

Les PA plus particulièrement mais non limitativement concernés par l'invention sont des protéines, des glycoprotéines, des peptides, des polysaccharides, des lipopolysaccharides, des oligo ou des polynucléotides, et des molécules organiques. Mais il peut aussi s'agir de produits cosmétiques ou de produits phytosanitaires, tels que des herbicides, des insecticides, des fongicides, etc.

Dans le domaine de la vectorisation des principes actifs notamment médicamenteux, il existe un besoin, dans beaucoup de cas :
- de les protéger contre la dégradation (hydrolyse, précipitation sur site, digestion enzymatique etc..) jusqu'à ce qu'ils atteignent leur site d'action,
- et/ou de contrôler leur vitesse de libération afin de maintenir un niveau thérapeutique sur une durée définie, soit
- et/ou de véhiculer (en les protégeant) au site d'action.

A ces fins, plusieurs types de polymères ont été étudiés et certains sont même disponibles commercialement. On peut citer par exemple les polymères du type polylactique, polylactique-glycolique, polyoxyethylène-oxypropylène, polyaminoacide ou encore polysaccharide. Ces polymères constituent des matières premières permettant de fabriquer, par exemple, des implants massiques, des microparticules, des nanoparticules, des vésicules, des micelles ou des gels. Outre le fait que ces polymères doivent être adaptés à la fabrication de tels systèmes, ils doivent également être biocompatible, non-toxiques, non-immunogènes, économiques et ils doivent pouvoir être facilement éliminés du corps et/ou biodégradables. Sur ce dernier aspect, il est de surcroît essentiel que la biodégradation dans l'organisme génère des produits non-toxiques.

A titre d'illustration de l'art antérieur concernant des polymères employés comme matières premières pour la réalisation de systèmes de vectorisation de PA, divers brevets ou demandes de brevet ou articles scientifiques sont évoqués ci-après.

Le brevet US-B-4,652,441 décrit des microcapsules de polylactide encapsulant hormone LH-RH. Ces microcapsules sont produites en préparant une émulsion eau-dans-huile-dans-eau et comprennent une couche interne aqueuse contenant l'hormone, une substance (gélatine) fixant cette dernière, une couche huileuse de polylactide, ainsi qu'une couche externe aqueuse (alcool polyvinylique). La libération du PA peut se faire sur une période de plus de deux semaines après injection sous-cutanée.

Le brevet US-B-6,153,193 décrit des compositions à base de micelles de poly(oxyéthylène)-poly(oxypropylène) amphiphiles, pour la vectorisation d'anti-cancéreux tel que l'adriamycine.

Akiyoshi et al. (J. Controlled Release 1998, 54, 313-320) décrivent des pullulans qui sont rendus hydrophobes par greffage de cholestérol et qui forment des nanoparticules dans l'eau. Ces nanoparticules aptes à se complexer de manière réversible avec l'insuline, forment des suspensions colloïdales stables.

Le brevet US-B-4,351,337 décrit des copolyaminoacides amphiphiles, à base de leucine et de glutamate, utilisables sous forme d'implants ou de microparticules pour la libération contrôlée de principes actifs. La libération de ces derniers peut se faire sur une durée très longue dépendant de la vitesse de dégradation du polymère.

Le brevet US-B-4,888,398 décrit des polymères à base de polyglutamate ou polyaspartate, et éventuellement polyleucine, avec des groupements pendants de type alkyloxycarbonylméthyle, placés de façon aléatoire sur la chaîne polyaminoacide. Ces polyaminoacides, greffés par des groupements latéraux e.g. méthoxycarbonylméthyle, sont utilisables sous forme d'implants biodégradables contenant un PA à libération prolongée.

Le brevet US-B-5,904,936 décrit des nanoparticules obtenues à partir d'un polymère bloc polyleucine-polyglutamate, aptes à former des suspensions colloïdales stables et capables de s'associer spontanément avec des protéines biologiquement actives sans les dénaturer. Ces dernières peuvent ensuite être libérées in vivo de manière contrôlée, sur une longue période.

Le brevet US-B-5,449,513 décrit des copolymères bloc amphiphile comprenant un bloc polyoxyéthylène et un bloc polyaminoacide, par exemple poly(bêta-benzyl-L-aspartate). Ces polymères polyoxyéthyléne-polybenzylaspartate forment des micelles qui sont aptes à encapsuler des molécules actives hydrophobes telles que l'adryamicine ou l'indométhacine.

La demande de brevet WO-A-99/61512 décrit des polylysines et des polyornithines fonctionnalisées par un groupe hydrophobe (acide palmitique relié à la polylysine ou ornithine) et un groupe hydrophile (polyoxyéthylène). Ces polymères, par exemple la polylysine greffée avec des chaînes polyoxyéthylène et palmitoyle forment en présence de cholestérol des vésicules capables d'encapsuler la doxorubicine ou l'ADN. Ces polymères à base de polylysines sont cationiques en milieu physiologique.

La demande de brevet WO-A-00/30618, de la demanderesse, décrit des polymères blocs ou aléatoires poly(glutamate de sodium)-poly(glutamate de méthyle, d'éthyle, d'hexa-décyle ou de dodécyle), aptes à former des suspensions colloïdales stables et capables de s'associer spontanément avec des protéines biologiquement actives sans les dénaturer. Ces dernières peuvent ensuite être libérées in vivo de manière contrôlée, sur une longue période. Ces copolyaminoacides amphiphiles sont modifiés par la présence d'une chaîne latérale alkyle hydrophobe. Ces groupements alkyles sont greffés de façon covalente sur le polymère via une fonction ester. Ces polymères sont anioniques en milieu physiologique.

La demande de brevet français non publiée FR N°02/07008 du 7/06/2002, décrit un polyglutamate portant des greffons à base d'alpha-tocophérol lié à un espaceur formé par un à quatre restes "acide aminé" et par exemple un reste leucine .

Ainsi, même s'il existe de très nombreuses solutions techniques dans l'art antérieur, développées et proposées pour la vectorisation des principes actifs médicamenteux, la réponse à l'ensemble des exigences est difficile à obtenir et demeure non satisfaisante. Plus spécifiquement, la conception d'un polyaminoacide greffé par des groupements hydrophobes, capable de former une suspension aqueuse colloïdale stable de particules de vectorisation propres à s'associer réversiblement à des principes actifs et peu onéreuse, est perfectible.

Dans ce contexte, l'un des objectifs essentiels de la présente invention est de fournir une nouvelle famille de polymères anioniques à pH physiologique animal, à base de polyglutamate et polyaspartate, qui représentent un perfectionnement par rapport à ceux décrits dans la demande de brevet WO-A-00/30618, notamment en termes de stabilité et de capacité d'absorption d'une protéine.

Un autre objectif essentiel de la présente invention est que ces polymères soient aptes à être utilisée pour la vectorisation de PA et permettent de satisfaire de manière optimale à toutes les spécifications du cahier des charges, à savoir notamment :
o capacité :
   - à former aisément et économiquement des suspensions colloïdales aqueuses stables,
   - à s'associer facilement avec de nombreux principes actifs,
   - et à libérer ces principes actifs in vivo,
o biocompatibilité,
o biodégradabilité,
o stabilité à l'hydrolyse.

Cet objectif parmi d'autres, est atteint par la présente invention qui concerne tout d'abord um polyaminoacide comprenant des unités aspartiques et/ou des unités glutamiques, dont certaines sont porteuses d'au moins un greffon, caractérisé en ce qu'au moins l'un de ces greffons :
- est relié à une unité aspartique ou glutamique de la chaîne principale, par l'intermédiaire d'un espaceur comprenant un ou plusieurs (oligo)aminoacide(s), formé(s) par une ou plusieurs unités "acides aminés" choisies parmi les unités "acides aminés" ayant un groupement alkyle ou aryle en alpha, de préférence parmi les unités "acides aminés" comprises dans le groupe comportant l'alanine, la valine, la leucine, l'isoleucine et la phénylalanine,
- et comprend au moins un groupement hydrophobe :
   ◆ comportant au moins 6 atomes de carbone, de préférence de 6 à 30 atomes (par exemple de 8-30 atomes de carbone),
   ◆ différent de l'alpha-tocophérol,
   ◆ et relié à l'espaceur par l'intermédiaire d'au moins une liaison ester.

Les inventeurs ont perfectionné les polyaminoacides connus en trouvant, de façon surprenante et inattendue, que le greffage sur ces polyminoacides de greffons comprenant des groupements hydrophobes issus de précurseurs de type alcool via un espaceur "(oligo)acide aminé" ayant une substitution en position alpha, améliorait grandement la stabilité de la liaison ester en milieu aqueux.

Il est du mérite de la demanderesse d'avoir eu l'idée de combiner, de façon tout à fait judicieuse et avantageuse, des polyaminoacides particuliers polyAsp et/ou polyGlu, biodégradables et anioniques avec des greffons reliés au squelette polyAsp et/ou polyGlu par un "acide aminé" ayant un groupement alkyle on aryle en alpha.

Ces nouveaux (co)polymères se sont avérés être particulièrement bien adaptés pour la vectorisation des protéines.

Au sens de l'invention le terme *«polyaminoacide»* couvre aussi bien les oligoaminoacides comprenant de 2 à 20 unités "acide aminé" que les polyaminoacides comprenant plus de 20 unités "acide aminé".

Avantageusement, l'oligoaminoacide ou les (oligo)aminoacides de tout ou partie des greffons est (sont) constitué(s) (chacun) par des unités "acide aminé" identiques entre elles.

Ces polymères présentent des propriétés surprenantes d'association et/ou d'encapsulation avec un ou plusieurs principes actifs, en comparaison avec des produits analogues. De plus, ils sont facilement dégradés, en présence d'enzymes, en catabolites/métabolites non toxiques (acides aminés).

Au sens de l'invention et dans tout le présent exposé, les termes "association" ou "associer" employés pour qualifier les relations entre un ou plusieurs principes actifs et les polyaminoacides, signifient en particulier que le ou les principes actifs sont liés au(x) polyaminoscide(s) notamment par une liaison faible, par exemple par liaison ionique et/ou par contact hydrophobe, et/ou sont encapsulés par le ou les polyaminoacides.

De préférence, les polyaminoacides selon la présente invention sont des oligoméres ou des homopolyméres comprenant des unités récurrentes acide glutamique ou aspartique ou des copolymères comprenant un mélange de ces deux types d'unités "acide aminé". Les unités considérées dans ces polymères sont des acides aminés ayant la configuration D ou L ou D/L et sont liées par leurs positions alpha ou gamma pour l'unité glutamate ou glutamique et alpha ou bêta pour l'unité aspartique ou aspartate.

Les unités "acide aminé" préférées de la chaîne polyaminoacide principale sont celles ayant la configuration L et une liaison de type alpha.

Les polyaminoacides selon l'invention répondent à la formule générale (I) suivante : 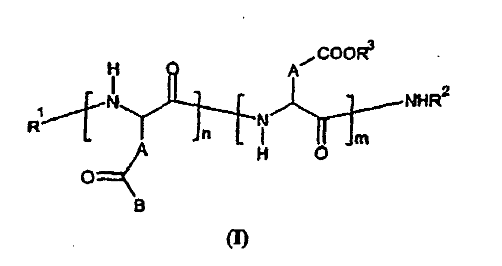 dans laquelle :
■ R¹ représente un H, un groupe acyle linéaire en C2 à C10 ou ramifié en C3 à C10, ou un pyroglutamate ;
■ R² représente un H, un alkyle linéaire en C2 à C10 ou ramifié en C3 à C10, benzyle, une unité "acide aminé" terminale ;
■ R³ est un H ou une entité cationique, de préférence sélectionnée dans le groupe comprenant :
   - les cations métalliques avantageusement choisis dans le sous-groupe comprenant : le sodium, le potassium, le calcium, le magnésium ;
   - les cations organiques avantageusement choisis dans le sous-groupe comprenant:
      - les cations à base d'amine,
      - les cations à base d'oligoamine,
      - les cations à base de polyamine (la polyéthylèneimine étant particulièrement préférée),
      - les cations à base d'acide(s) aminé(s) avantageusement choisis dans la classe comprenant les cations à base de lysine on d'arginine,
   - ou les polyaminoacides cationiques avantageusement choisis dans le sous-groupe comprenant la polylysine ou l'oligolysine ;
■ les n groupements B représentent chacun indépendamment les uns des autres un radical monovalent de formule suivante : 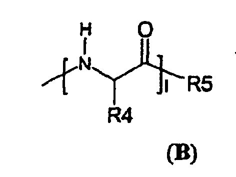 dans laquelle :
   - R⁴ représente un méthyle(alanine), isopropyle (valine), isobutyle (leucine), secbutyle (isoleucine), benzyle (phenylalanine), les acides aminés mentionnés entre parenthèses sont ceux qui correspondant à l'unité "acide aminé" formée lorsque R⁴ représente l'alkyle considéré ;
   - R⁵ représente un groupement hydrophobe comportant 6 à 30 (par exemple 8 à 30) atomes de carbone et de préférence lié au polymère (plus précisément à l'espaceur [-NH-CHR4-CO-]ₗ par l'intermédiaire d'une fonction ester ;
   - 1 varie de 1 à 6 ;
■ A représente indépendamment un -CH₂- (unité aspartique) ou -CH₂-CH₂-(unité glutamique) ;
■ n/(n+m) est défini comme le taux de greffage molaire et varie de 0,5 à 100 % molaire ;
■ n + m varie de 3 à 1000, de préférence entre 30 et 300.

De manière plus préférée encore, on utilise comme espaceur dans tout ou partie des greffons, d'un seul acide aminé (l=1 dans la formule **B** supra) fonctionnalisé par un groupement hydrophobe. Il a pu être constaté que cela permet notamment d'améliorer les taux d'association des polyaminoacides avec des principes actifs.

Selon une caractéristique remarquable de l'invention, tout ou partie des groupements hydrophobes R⁵ des greffons sont choisis de façon indépendante dans le groupe de radicaux comportant:
■ un alcoxy linéaire ou ramifié comportant de 6 à 30 atomes de carbone et pouvant comporter au moins un hétéroatome (de préférence O et/ou N et/ou S) et/ou au moins une insaturation,
■ un alcoxy comportant 6 à 30 atomes de carbone et ayant un ou plusieurs carbocycles annelés et contenant éventuellement au moins une insaturation et/ou au moins un hétéroatome (de préférence O et/ou N et/ou S),
■ un alcoxyaryle ou un aryloxyalkyle de 7 à 30 atomes de carbone et pouvant comporter au moins une insaturation et/ou au moins un hétéroatome (de préférence O et/ou N et/ou S).

Plus avantageusement encore, le groupement hydrophobe du greffon est issu d'un précurseur alcoolique est choisi dans le groupe comprenant: l'octanol, le dodécanol, le tétradécanol, l'hexadécanol, l'octadécanol, l'oleylalcool ou le cholestérol.

S'agissant de l'unité "acide aminé" du greffon, on retiendra plus spécialement dans le cadre de invention une unité "acide aminé" issue du groupe comprenant : la L-leucine, la L-valine ou la L-phénylalanine.

Selon un premier mode de réalisation de l'invention, les chaînes principales des polyaminoacides sont des homopolymères d'alpha-L-glutamate ou d'acide alpha-L-glutamique.

Selon un deuxième mode de réalisation de l'invention, les chaînes principales des polyaminoacides sont des homopolymères d'alpha-L-aspartate ou d'alpha-L-aspartique.

Selon un troisième mode de réalisation de l'invention, les chaînes principales des polyaminoacides sont des copolymères d'alpha-L-aspartate/alpha-L-glutamate ou d'acide alpha-L-aspartique/acide alpha-L-glutamique.

Avantageusement, la distribution des unités aspartiques et/ou glutamiques de la chaîne polyaminoacide principale est telle que les polymères ainsi constitués sont soit aléatoires, soit de type bloc, soit de type multibloc.

Selon un autre mode de définition, les polyaminoacides selon invention ont une masse molaire qui se situe entre 2 000 et 100 000 g/mole, et de préférence entre 5 000 et 40 000 g/mole.

Il est par ailleurs préférable que le taux de greffage molaire en motif hydrophobe des polyaminoacides selon l'invention, soit compris entre 2 et 70 %, et de préférence entre 5 et 40%.

De manière remarquable, les polyaminoacides de l'invention sont susceptibles d'être utilisés de plusieurs façons selon le taux de greffage. Les méthodes de mise en forme d'un polymère pour l'encapsulation d'un principe actif sous les diverses formes visées par l'invention sont connues de l'homme de l'art. Pour plus de détails, on peut se référer, par exemple à ces quelques références particulièrement pertinentes :
*"*Microspheres, Microcapsules and Liposomes ; vol 1. Preparation and chemical applications" Ed. R. Arshady, Citus Books 1999. ISBN : 0-9532187-1-6. *"*Sustained-Release Injectable Products" Ed. J. Senior et M. Radomsky, Interpharm Press 2000. ISBN : 1-57491-101-5.
*"*Colloidal Drug Delivery Systems" Ed. J. Kreuter, Marcel Dekker, Inc. 1994. ISBN: 0-8247-9214-9.
*"*Handbook of Pharmaceutical Controlled Release Technology" Ed. D.L. Wise, Marcel Dekker, Inc. 2000. ISBN : 0-8247-0369-3.

Les polyaminoacides sont en outre extrêmement intéressants, du fait qu'à un taux de greffage relativement faible de l'ordre de 3 à 30, ils se dispersent dans l'eau à pH 7,4 (par exemple avec un tampon phosphate) pour donner des solutions ou des suspensions colloïdales ou des gels, en fonction de la concentration de polymère et du taux de greffage. De plus, les polyaminoacides (sous forme de particules ou non), peuvent encapsuler ou s'associer aisément avec des principes actifs tels que des protéines, peptides ou petites molécules. La mise en forme préférée est celle décrite dans la demande de brevet WO-A-00/30618 de la demanderesse et qui consiste à disperser le polymère dans l'eau et à incuber la solution en présence d'un PA. Cette solution colloïdale de particules de vectorisation constituées des polyaminoacides selon invention, peut ensuite être filtrée sous 0,2 µm puis directement injectée à un patient.

Cette forme particulaire selon la demande de brevet WO-A-00/30618 est notamment envisageable en l'espèce, au-delà de 30 % de taux de greffage et selon le greffon choisi. Le polymère peut alors former des microparticules capables d'associer ou d'encapsuler des PA. Dans ce contexte, la mise en forme des microparticules peut se faire en co-solubilisant le PA et le polymère dans un solvant organique approprié puis le mélange précipité dans l'eau. Les particules sont ensuite récupérées par filtration et peuvent ensuite être utilisées pour une administration par voie orale (sous forme de gélule, sous forme compactée et/ou enrobée ou bien encore sous forme dispersée dans une huile) ou par voie parentérale après redispersion dans l'eau.

A des taux supérieurs à 50 % de greffage, la redispersion du polymère en phase aqueuse devient plus difficile du fait de la quantité plus faible des fonctions carboxylate ionisables et le polymère précipite. Dans ce cas, le polymère peut être solubilisé dans un solvant biocompatible tel que la N-méthylpyrrolidone ou une huile appropriée telle que le MygliolⓇ puis injecté en intramusculaire ou sous-cutanée ou dans une tumeur. La diffusion du solvant ou de l'huile conduit à la précipitation du polymère sur le site d'injection et forme ainsi un dépôt Ces dépôts assurent ensuite une libération contrôlée par diffusion et/ou par érosion et/ou par dégradation hydrolytique ou enzymatique du polymère.

Indépendamment du fait que la forme microparticulaire du polyaminoacide selon l'invention est préférée, les polymères de l'invention, sous forme neutre ou ionisée, sont de façon plus générale, utilisables seuls ou dans une composition liquide, solide ou gel et dans un milieu aqueux ou organique.

Il convient de comprendre que le polymère à base de polyaminoacides contient des fonctions carboxyliques qui sont soit neutres (forme COOH), soit ionisées (anion COO⁻), selon le pH et la composition. Pour cette raison, la solubilité dans une phase aqueuse est directement fonction du taux de COOH libre du polymère (non greffé par le motif hydrophobe) et du pH. En solution aqueuse, le contre-cation peut être un cation métallique tel que le sodium, le calcium ou le magnésium, ou un cation organique tel que la triéthanolamine, la tris(hydroxyméthyl)-aminoméihane ou une polyamine tel que la polyéthylèneimine.

Les polymères de l'invention sont par exemple obtenus par des méthodes connues de l'homme de l'art. Les polyaminoacides statistiques peuvent être obtenus par greffage du greffon hydrophobe, préalablement fonctionnalisé par l'acide aminé espaceur, directement sur le polymère par une réaction classique de couplage. Les polyaminoacides blocs ou multiblocs peuvent être obtenus par polymérisation séquentielle des anhydrides de N-carboxy-aminoacides (NCA) correspondants.

On prépare par exemple un polyaminoacide, homopolyglutamate, homopoly-aspartate ou un copolymère glutamate/aspartate, bloc, multibloc ou aléatoire selon des méthodes classiques.

Pour l'obtention de polyaminoacide de type alpha, la technique la plus courante est basée sur la polymérisation d'anhydrides de N-carboxy-arninoacides (NCA), décrites, par exemple, dans l'article *"*Biopolymers, 1976,15, 1869 et dans l'ouvrage de H.R. Kricheldorf "alpha-Aminoacid-N-carboxy Anhydride and related Heterocycles" Springer Verlag (1987). Les dérivés d'NCA sont de préférence des dérivés NCA-O-Me, NCA-O- et/ou NCA-O-Bz (Me = Méthyl, Et = Ethyle et Bz = Benzyle). Les polymères sont ensuite hydrolysés dans des conditions appropriées pour obtenir le polymère sous sa forme acide. Ces méthodes sont inspirées de la description donnée dans le brevet FR-A-2 801 226 de la demanderesse. Un certain nombre de polymères utilisables selon l'invention, par exemple, de type poly(acide alpha-L-aspartique), poly(acide alpha-L-glutamique), poly(acide alpha-D-glutamique) et poly(acide gamma-L-glutamique) de masses variables sont disponibles commercialement. Le polyaspartique de type alpha-bêta est obtenu par condensation de l'acide aspartique (pour obtenir un polysuccinimide) suivie d'une hydrolyse basique (cf. Tomida et aL Polymer 1997, 38, 4733-36).

Le couplage du greffon avec une fonction acide du polymère est réalisé aisément par réaction du polyaminoacide en présence d'un carbodiimide comme agent de couplage et optionnellement, un catalyseur tel que le 4-dimethylaminopyridine et dans un solvant approprié tel que la diméthylformamide (DMF), la N-méthyl pyrrolidone (NMP) ou la diméthylsulfoxide (DMSO). Le carbodiimide est par exemple, le dicyclohexylcarbo-diimide ou le diisopropylcarbodiimide. Le taux de greffage est contrôlé chimiquement par la stoechiométrie des constituants et réactifs ou le temps de réaction. Les greffons hydrophobes fonctionnalisés par un acide aminé sont obtenus par couplage peptidique classique ou par condensation directe par catalyse acide. Ces techniques sont bien connues de l'homme de l'art.

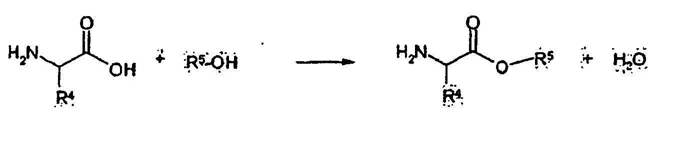

Pour la synthèse de copolymère bloc ou multibloc, on utilise des dérivés NCA préalablement synthétisé avec le greffon hydrophobe. Le schéma de synthèse est le suivant.

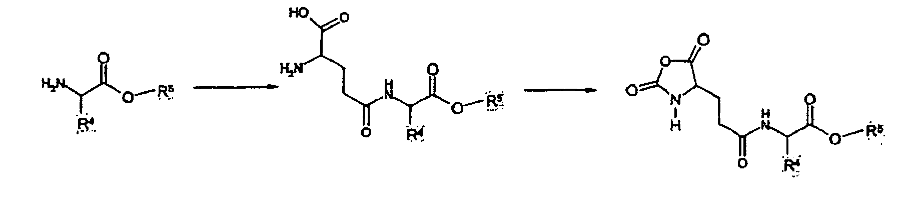

Préférentiellement, le dérivé NCA-hydrophobe est copolymérisé avec le NCA-O-Benzyl puis on enlève par hydrolyse sélectivement les groupements benzyliques.

Selon un autre de ses aspects, l'invention vise une composition pharmaceutique, cosmétique, diététique ou phytosanitaire comprenant au moins un polyaminoacide tel que défini ci-dessus et éventuellement au moins un principe actif, qui peut être thérapeutique, cosmétique, diététique ou phytosanitaire.

Suivant une disposition intéressante de l'invention, le principe actif est associé au(x) polyaminoacide(s) par une ou plusieurs liaisons autre(s) qu'une (ou que des) liaison(s) chimique(s) covalente(s).

Les techniques d'association d'un ou de plusieurs PA aux polyaminoacides greffés selon l'invention, sont décrites notamment dans la demande de brevet WO-A-00/30618. Elles consistent à incorporer au moins un principe actif dans le milieu liquide contenant des particules PV, de manière à obtenir une suspension colloïdale de PV chargées en ou associées avec un ou plusieurs principe(s) actif(s) PA. Cette incorporation, qui conduit à un piégeage de PA par les PV, peut être réalisée de la manière suivante :
- mise en solution aqueuse de PA, puis ajout des PV, soit sous forme de suspension colloïdale, soit sous forme de PV isolées (lyophilisat ou précipitat) ;
- ou ajout de PA, soit en solution, soit à l'état pur ou préformulé, à une suspension colloïdale de particules PV, éventuellement préparée extemporanément par la dispersion de PV sèches dans un solvant approprié, tel que l'eau.

De préférence, le principe actif est une protéine, une glycoprotéine, une protéine liée à une ou plusieurs chaînes polyalkylèneglycol (de préférence PolyÉthylèneGlycol (PEG) : "protéine-PBGylée"), un polysaccharide, un liposaccharide, un oligonucléotide, un polynucléotide ou un peptide.

Selon une variante, le principe actif est une "petite" molécule organique hydrophobe, hydrophile ou amphiphile.

Au sens du présent exposé, une "petite" molécule est notamment une petite molécule non protéinique.

Comme exemples de PA susceptibles d'être associés aux polyaminoacides selon l'invention, qu'ils soient ou non sous forme de (nano ou micro)particules, on peut citer :
o les protéines telles que l'insuline, les interférions, les hormones de croissance, les interleukines, l'érythropoïétine ou les cytokines ;
o les peptides telles que la leuprolide ou la cyclosporine ;
o les petites molécules telles que celles appartenant à la famille des anthracyclines, des taxoïdes ou des camptothécines ;
o et leurs mélanges.

Selon un mode de réalisation, la composition de l'invention est sous forme d'un gel, d'une solution, d'une suspension, d'une émulsion, de micelles, de nanoparticules, de microparticules, d'un implant, d'une poudre ou d'un film.

Suivant l'une de ses formes particulièrement préférées, la composition, chargée ou non en principe actif(s), est une suspension colloïdale stable de nanoparticules et/ou de microparticules et/ou de micelles de polyaminoacides, dans une phase aqueuse.

Selon une autre mode de réalisation, la composition de l'invention est sous forme de solution dans un solvant biocompatible et peut être injectée par voie sous-cutanée, intramusculaire ou dans une tumeur.

La composition selon l'invention, dès lors qu'elle est pharmaceutique, peut étre administrée par voie orale, parentérale, nasale, vaginale, oculaire, sous-cutanée, intraveineuse, intramusculaire, intradermique, intrapéritonéale, intracérébrale ou buccale.

Il est également envisageable que la composition soit sous forme de solution dans un solvant biocompatible, susceptible d'être injectée en sous-cutané, intramusculaire ou dans une tumeur.

Selon une autre variante, la composition selon l'invention est formulée de telle sorte qu'elle soit apte à former un dépôt sur la site d'injection.

L'invention vise aussi des compositions qui comprennent des polyaminoacides selon l'invention et des principes actifs et qui sont susceptibles d'être utilisées pour la préparation :
- de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkyléneglycol {par exemple PotyÉthylèneGlycol (PEG), on parle alors de protéines "PEGylées"}, des peptides, des polysaccharides, des liposaccharides, des oligonucléotides, des polynucléotides et des molécules organiques non protéiniques hydrophobes,
- hydrophiles ou amphiphiles;
- et/ou des nutriment ;
- et/ou de produits cosmétiques ou phytosanitaires.

Selon encore un autre de ses aspects, invention vise un procédé de préparation:
- de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyÉthylèneGlycol (PEG), on parle alors de protéines "PEGylées"}, des peptides, des polysaccharides, des liposaccharides, des oligonucléotides, des polynucléotides et des molécules organiques non protéiniques hydrophobes, hydrophiles ou amphiphiles ;
- et/ou des nutriments ;
- et/ou de produits cosmétiques ou phytosanitaires ;
ce procédé étant caractérisé en ce qu'il consiste essentiellement à mettre en oeuvre au moins un polyaminoacide tel que défini ci-dessus et/ou la composition elle aussi décrite supra.

L'invention concerne également une méthode de traitement thérapeutique consistant essentiellement à administrer la composition telle que décrite dans le présent exposé, par voie orale, parentérale, nasale, vaginale, oculaire, sous-cutanée, intraveineuse, intramusculaire, intradermique, intrapéritonéale, intracérébrale ou buccale.

Suivant une variante particulière de l'invention, cette méthode de traitement thérapeutique consiste essentiellement à administrer la composition telle que décrite supra sous forme de solution dans un solvant biocompatible puis de l'injecter en sous-cutané, intramusculaire ou dans une tumeur, de préférence de manière à ce qu'elle forme un dépôt sur le site d'injection.

L'invention sera mieux comprise et ses avantages et variantes de mise en oeuvre ressortiront bien des exemples qui suivent et qui décrivent la synthèse des polyaminoacides greffés par un groupement hydrophobe, leur transformation en système de vectorisation de PA (suspension colloïdale aqueuse stable) et la démonstration de la capacité d'un tel système de s'associer à une protéine pour former des compositions pharmaceutiques.

### Exemple 1 : Préparation du polymère P1

### Synthèse d'un polyglutamate greffé avec un greffon LeuOC12

### 1/ Structure du greffon:

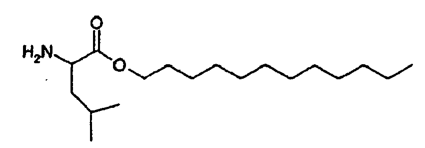

Ce produit est synthétisé à partir de la L-leucine et de la dodécanol par condensation en présence d'un acide, selon un procédé décrit dans le brevet US-A-4,826,818.

### 2/ Synthèse du polymère :

Le polymère (acide alpha-L-polyglutamique) (de masse équivalente à environ 12 000 g/mole par rapport à un standard en polyoxyéthylène) est obtenu par polymérisation de monomères constitués par des dérivés N-CarboxyAnhydride de glutamate de méthyle : NCAGluOMe. Cette polymérisation est suivie d'une hydrolyse comme décrit dans la demande de brevet FR-A-2 801 226. 18 g du polymère est ensuite mis en solution dans 360 ml de DiMéthylFormamide (DMF) en chauffant à 80°C pendant 2 heures. Une fois le polymère solubilisé, on laisse revenir la température à 25 °C et on ajoute successivement 5,0 g du greffon LeuOC12 préalablement solubilisé dans 9 ml de DMF, 0,5 g de 4-diméthylaminopyridine préalablement solubilisé dans 5 ml de DMF et 3,16 g de diisopropylcarbodiimide. Après 6 heures à 25°C sous agitation, le milieu réactionnel est versé dans 720 ml d'eau contenant 15 % de chlorure de sodium et d'acide chlorhydrique (pH 2). Le polymère précipité est ensuite récupéré par filtration, lavé par de l'acide chlorhydrique 0,1 N puis par de l'éther diisopropylique. Le polymère est ensuite séché à l'étuve sous vide à 40°C. On obtient un rendement de l'ordre de 90 %. Le taux de greffage estimé par RMN du proton est d'environ 11,6 %.

### Exemple 2 : Préparation des polymères P2 à P5, C1 et C2

Les polymères P2 à P5 et les exemples comparatifs C1 et C2 sont synthétisés selon le même protocole que pour le polymère P1. Pour le greffon ValChol, nous avons réalisé un couplage du cholestérol avec de la Bocvaline en présence du diisopropylcarbodiimide suivi de la déprotection de l'amine en milieu acide (voir par exemple l'ouvrage « Principles of peptide synthesis » par Bodanszky, Springer-Verlag 1984).

### Exemple 3 : Caractéristiques des polymères

**Tableau 1**

| Polymère | Greffon | Structure du greffon[1] | % molaire du greffon [2] | Mn g/mole [3] |
|---|---|---|---|---|
| P1 | L-LeuOC12 | 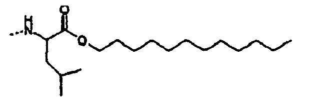 | 11,6 | 13500 |
| P2 | L-LeuOC8 | 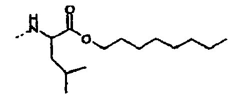 | 14,7 | 14000 |
| P3 | L-ValOC12 | 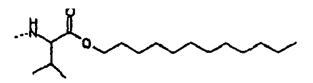 | 11,8 | 15700 |
| P4 | L-PheOC12 | 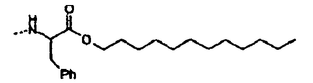 | 10,4 | 11000 |
| P5 | L-ValCHOL | 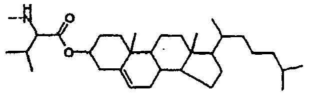 | 5,5 | 13300 |
| C1 | OC12 | 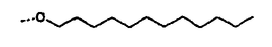 | 15,3 | 10500 |
| C2 | (L-Leu)₃NH₂ | 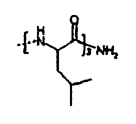 | 21,0 | - |

| | | | | |
|---|---|---|---|---|
| [1] La liaison en pointillé représente la liaison au polymère. [2] Le % de greffage molaire est estimé par la RMN du proton du polymère solubilisé dans l'acide trifluoroacétique deutérié. [3] Mn est la masse molaire en nombre mesurée par la chromatographie d'exclusion stérique en éluant avec un mélange de tampon PBS (pH 7,4) et d'acétonitrile et est donnée en référence à un polyoxyéthylène utilisé comme étalon. | | | | |

### Exemple 4 : Etude d'association avec l'insuline

On prépare une solution aqueuse contenant 10 mg de polymère par millilitre à pH 7,4 et 200 UI d'insuline (7,4 mg). On laisse incuber les solutions pendant deux heures à température ambiante et on sépare l'insuline libre de l'insuline associée par ultrafiltration (seuil à 100 KDa, 15 minutes sous 10000G à 18 °C). L'insuline libre récupérée dans le filtrat est ensuite dosée par CLHP (Chromatographie Liquide Haute Performance) et l'on déduit la quantité d'insuline associée. Les résultats sont donnés dans le tableau 2 ci-dessous.

**Tableau 2**

| Polymère | % association |
|---|---|
| P1 | 97% |
| P2 | 92% |
| P3 | 97 % |
| P5 | 98% |
| C1 | 75% |
| C2 | 55% |

Les résultats démontrent que les polymères de l'invention présentent des taux d'association avec l'insuline supérieur à ceux n'ayant pas d'espaceur "acide aminé" (C1) ou ayant une séquence d'acide aminé hydrophobe mais pas de groupement hydrophobe de type alkyle linéaire ou polycyclique (C2).

### Exemple 5 : Etude de la stabilité des polymères en milieu aqueux.

Les polymères sont solubilisés à 20 mg/mL d'eau en ajustant le pH à 6,0 et les solutions limpides sont ensuite placées dans des étuves thermostatés à 25 ou 37°C pendant une semaine. La gamme de pH utile dans les applications visées est souvent comprise entre 6 et 7,4. Nous avons choisi un pH de 6 pour cette étude afin d'accélérer la cinétique de dégradation. L'analyse des polymères par diverses techniques montre que la seule voie de dégradation du polymère dans ces conditions, est l'hydrolyse des greffons esters. Le taux d'hydrolyse des polymères P1, P3 et C1 en fonction du temps est donné dans le tableau 3 suivant.

**Tableau 3**

| Polymère | % d'hydrolyse | % d'hydrolyse |
|---|---|---|
| | 1 semaine à 25°C | 1 semaine à 37°C |
| P1 | 0,94% | 1,91% |
| P3 | 0,01% | 0,30% |
| C1 | 1,63% | 4,31 % |

Ces résultats montrent que P1 et P3, en plus d'avoir une capacité d'adsorption plus élevée avec l'insuline, sont plus stables à l'hydrolyse.

## Revendications

1. Polyaminoacide comprenant des unités aspartiques et/ou des unités glutamiques, répondant à la formule (1) suivante : 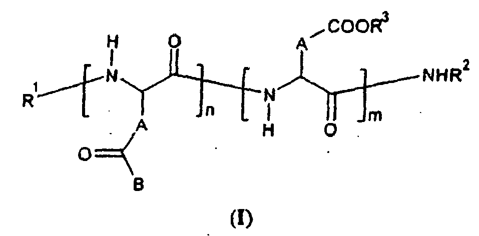 dans laquelle :
■ R¹ représente un H, un groupe acyle linéaire en C2 à C10 ou ramifié en C3 à C10, ou un pyroglutamate ;
■ R² représente un H, un alkyle linéaire en C2 à C10 ou ramifié en C3 à C10, benzyle, une unité "acide aminé" terminale ;
■ R³ est un H ou une entité cationique,
■ les n groupements B représentent chacun indépendamment les uns des autres un radical monovalent de formule suivante : 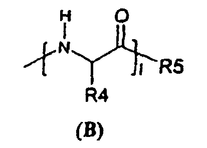 dans laquelle :
- R4 représente un méthyle, isopropyle, isobutyle , secbutyle, benzyle ;
- R5 représente un groupement hydrophobe comportant de 6 à 30 atomes de carbone ;
- l varie de 1 à 6 ;
■ A représente indépendamment un -CH₂- ou -CH₂-CH₂- ;
■ n/(n + m) est défini comme le taux de greffage molaire et varie de 0,5 à 100 % molaire ;
■ n + m varie de 3 à 1000, de préférence entre 30 et 300.

2. Polyaminoacide selon la revendication 1, **caractérisé en ce que** dans R³, l'entité cationique est sélectionnée parmi :
- les cations métalliques,
- les cations organiques,
- et les polyaminoacides cationiques.

3. Polyaminoacide selon la revendication 1 ou 2, **caractérisé en ce que** dans R³. l'entité cationique est sélectionnée parmi :
- les cations métalliques choisis parmi le sodium, le potassium, le calcium et le magnésium,
- les cations organiques choisis parmi:
• les cations à base d'amine,
• les cations à base d'oligoamine,
• les cations à base de polyamine,
• les cations à base d'acide(s) aminé(s),
- et les polyaminoacides cationiques choisis parmi la polylysine et l'oligolysine.

4. Polyaminoacide selon la revendication 1, **caractérisé en ce que** tout ou partie des groupements hydrophobes R⁵ sont choisis de façon indépendante parmi les radicaux comportant :
• un alcoxy linéaire ou ramifié comportant de 6 à 30 atomes de carbone et pouvant comporter au moins un hétéroatome (de préférence O et/ou N et/ou S) et/ou au moins une insaturation,
• un alcoxy comportant 6 à 30 atomes de carbone et ayant un ou plusieurs carbocycles annelés et contenant éventuellement au moins une insaturation et/ou au moins un hétéroatome (de préférence O et/ou N et/ou S),
• un alcoxyaryle ou un aryloxyalkyle de 7 à 30 atomes de carbone et pouvant comporter au moins une insaturation et/ou au moins un hétéroatome (de préférence O et/ou N et/ou S).

5. Polyaminoacide selon l'une quelconque des revendications 1 à 4, **caractérisé en ce que** le groupement hydrophobe du greffon est issu d'un précurseur alcoolique choisi parmi: l'octanol, le dodécanol, le tétradécanol, l'hexadécanol, l'octadécanol, l'oleylalcool ou le cholestérol.

6. Polyaminoacide selon l'une quelconque des revendications 1 à 4, **caractérisé en ce que** l'unité "acide aminé" du greffon est choisie parmi: la L-leucine, la L-valine ou la L-phènylalanine et leurs mélanges.

7. Polyaminoacide selon l'une quelconque des revendications 1 à 6, **caractérisé en ce que** sa chaîne principale est constituée d'un homopolymère d'alpha-L-glutamate ou d'acide alpha-L-glutamique.

8. Polyaminoacide selon l'une quelconque des revendications 1 à 6, **caractérisé en ce que** sa chaîne principale est constituée d'un homopolymère d'alpha-L-aspartate ou d'acide alpha-L-aspartique.

9. Polyaminoacide selon l'une quelconque des revendications 1 à 6, **caractérisé en ce que** sa chaîne principale est constituée d'un copolymère d'alpha-L-aspartate/alpha-L-glutamate ou d'acide alpha-L-aspartique/acide alpha-L-glutamique.

10. Polyaminoacide selon l'une quelconque des revendications 1 à 9, **caractérisé en ce que** la distribution des unités aspartiques et/ou glutamiques porteuses de greffons est telle que les polymères ainsi constitués sont soit aléatoires, soit de type bloc, soit de type multibloc.

11. Polyaminoacide selon l'une quelconque des revendications 1 à 10, **caractérisé en ce que** sa masse molaire se situe entre 2 000 et 100 000 g/mole, et de préférence entre 5 000 et 40 000 g/mole.

12. Polyaminoacide selon l'une quelconque des revendications 1 à 11, **caractérisé en ce que** le taux de greffage molaire se situe entre 2 et 70 %, et de préférence entre 5 et 40 %.

13. Composition pharmaceutique, cosmétique, diététique ou phytosanitaire comprenant au moins un polyaminoacide selon l'une quelconque des revendications 1 à 12.

14. Composition selon la revendication 13, **caractérisée en ce qu'**elle comprend au moins un principe actif.

15. Composition selon la revendication 14, **caractérisée en ce que** le principe actif est associé au(x) polyaminoacide(s) par une ou plusieurs liaisons autre(s) qu'une (ou que des) liaison(s) chimique(s) covalente(s).

16. Composition selon la revendication 14 ou 15, **caractérisée en ce que** le principe actif est une protéine, une glycoprotéine, une protéine liée à une ou plusieurs chaînes polyalkylèneglycol {de préférence PolyEthylèneGlycol (PEG) }, un polysaccharide, un liposaccharide, un oligonucléotide, un polynucléotide ou un peptide.

17. Composition selon la revendication 14 ou 15, **caractérisée en ce que** le principe actif est une molécule organique hydrophobe, hydrophile ou amphiphile.

18. Composition selon l'une quelconque des revendications 13 à 17, **caractérisée en ce qu'**elle est une suspension colloïdale de nanoparticules et/ou de microparticules et/ou de micelles du ou des polyaminoacides, dans une phase aqueuse.

19. Composition selon l'une quelconque des revendications 13 à 18, **caractérisée en ce qu'**elle est sous forme d'une solution, d'un gel, d'une suspension, d'une émulsion, de micelles, de nanoparticules, de microparticules, d'un implant, d'une poudre ou d'un film.

20. Composition selon l'une quelconque des revendications 13 à 19, **caractérisée en ce qu'**elle est sous une forme adaptée à l'administration par voie orale, parentérale, nasale, vaginale, oculaire, sous-cutanée, intraveineuse, intramusculaire, intradermique, intrapéritonéale, intracérébrale ou buccale.

21. Composition selon l'une quelconque des revendications 13 à 20, **caractérisée en ce qu'**elle est sous forme de solution dans un solvant biocompatible et **en ce qu'**elle est sous une forme adaptée à l'injection par voie sous-cutanée, intramusculaire ou dans une tumeur.

22. Composition selon l'une quelconque des revendications 13 à 21, **caractérisée en ce qu'**elle est injectable et **en ce qu'**elle est apte à former un dépôt sur le site d'injection.

23. Composition selon l'une quelconque des revendications 13 à 22, **caractérisée en ce qu'**elle est destinée à la préparation :
• de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyEthylène-Glycol (PEG), }, des peptides, des polysaccharides, des liposaccharides, des oligonucléotides, des polynucléotides et des molécules organiques non proteiniques hydrophobes, hydrophiles ou amphiphiles;
• et/ou des nutriments ;
• et/ou de produits cosmétiques ou phytosanitaires.

24. Procédé de préparation :
• de médicaments, en particulier pour administration orale, nasale, vaginale, oculaire, sous-cutanée, intraveineuse, intramusculaire, intradermique, intrapéritonéale ou intracérébrale, les principes actifs de ces médicaments pouvant être, notamment, des protéines, des glycoprotéines, des protéines liées à une ou plusieurs chaînes polyalkylèneglycol {par exemple PolyEthylène-Glycol (PEG) }, des peptides, des polysaccharides, des liposaccharides, des oligonucléotides, des polynucléotides et des molécules organiques non protéiniques hydrophobes, hydrophiles ou amphiphiles ;
• et/ou des nutriments ;
• et/ou de produits cosmétiques ou phytosanitaires ;
**caractérisé en ce qu'**il consiste essentiellement à mettre en oeuvre au moins un polyaminoacide selon l'une quelconque des revendications 1 à 12 et/ou la composition selon l'une quelconque des revendications 13 à 22.

## Claims

1. Polyamino acid comprising aspartic units and/or glutamic units, having the general formula (I) below: 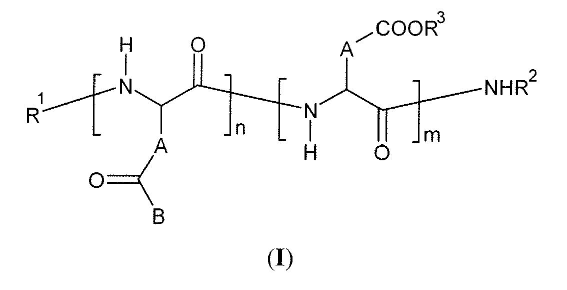 in which:
■ R¹ is an H, a linear C2 to C10 or branched C3 to C10 acyl group, or a pyroglutamate;
■ R² is an H, a linear C2 to C10 or branched C3 to C10 alkyl, a benzyl or a terminal "amino acid" unit;
■ R³ is an H or a cationic entity,
■ the n groups B each independently of one another are a monovalent radical of the formula below: 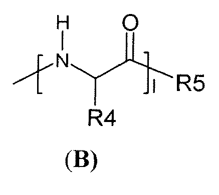 in which:
- R⁴ is a methyl, an isopropyl, an isobutyl, a sec-butyl or a benzyl;
- R⁵ is a hydrophobic group containing from 6 to 30 carbon atoms;
- 1 varies from 1 to 6;
■ A independently is -CH₂- or -CH₂-CH₂-;
■ n/(n + m) is defined as the molar grafting rate and varies from 0.5 to 100 mol%;
■ n + m varies from 3 to 1000, preferably between 30 and 300.

2. Polyamino acid according to claim 1, **characterized in that** the cationic entity in R³ is selected from:
- metal cations;
- organic cations;
- and cationic polyamino acids.

3. Polyamino acid according to claim 1 or 2, **characterized in that** the cationic entity in R³ is selected from:
- metal cations selected from sodium, potassium, calcium and magnesium;
- organic cations selected from:
• cations based on amine,
• cations based on oligoamine,
• cations based on polyamine,
• cations based on amino acid(s),
- and cationic polyamino acids selected from polylysine and oligolysine.

4. Polyamino acid according to claim 1, **characterized in that** all or some of the hydrophobic groups R⁵ are independently selected from the radicals comprising:
■ a linear or branched alkoxy containing from 6 to 30 carbon atoms and capable of containing at least one heteroatom (preferably O and/or N and/or S) and/or at least one unsaturation,
■ an alkoxy containing 6 to 30 carbon atoms, having one or more fused carbocyclic rings and optionally containing at least one unsaturation and/or at least one heteroatom (preferably O and/or N and/or S), and
■ an alkoxyaryl or aryloxyalkyl having 7 to 30 carbon atoms and capable of containing at least one unsaturation and/or at least one heteroatom (preferably O and/or N and/or S).

5. Polyamino acid according to any one of claims 1 to 4, **characterized in that** the hydrophobic group of the graft is derived from an alcohol precursor selected from: octanol, dodecanol, tetradecanol, hexadecanol, octadecanol, oleyl alcohol and cholesterol.

6. Polyamino acid according to any one of claims 1 to 4, **characterized in that** the "amino acid" unit of the graft is selected from: L-leucine, L-valine, L-phenylalanine and mixtures thereof.

7. Polyamino acid according to any one of claims 1 to 6, **characterized in that** its main chain consists of an alpha-L-glutamate or alpha-L-glutamic acid homopolymer.

8. Polyamino acid according to any one of claims 1 to 6, **characterized in that** its main chain consists of an alpha-L-aspartate or alpha-L-aspartic acid homopolymer.

9. Polyamino acid according to any one of claims 1 to 6, **characterized in that** its main chain consists of an alpha-L-aspartate/alpha-L-glutamate or an alpha-L-aspartic acid/alpha-L-glutamic acid copolymer.

10. Polyamino acid according to any one of claims 1 to 9, **characterized in that** the distribution of the aspartic and/or glutamic units carrying grafts is such that the resulting polymers are either random, of block type or of multiblock type.

11. Polyamino acid according to any one of claims 1 to 10, **characterized in that** its molar weight is between 2,000 and 100,000 g/mol, preferably between 5,000 and 40,000 g/mol.

12. Polyamino acid according to any one of claims 1 to 11, **characterized in that** the molar grafting rate is between 2 and 70%, preferably between 5 and 40%.

13. Pharmaceutical, cosmetic, dietetic or phytosanitary composition comprising at least one polyamino acid according to any one of claims 1 to 12.

14. Composition according to claim 13, **characterized in that** it comprises at least one active principle.

15. Composition according to claim 14, **characterized in that** the active principle is associated with the polyamino acid(s) by one or more bonds other than covalent chemical bonds.

16. Composition according to claim 14 or 15, **characterized in that** the active principle is a protein, a glycoprotein, a protein bonded to one or more polyalkylene glycol chains {preferably polyethylene glycol (PEG)}, a polysaccharide, a liposaccharide, an oligonucleotide, a polynucleotide or a peptide.

17. Composition according to claim 14 or 15, **characterized in that** the active principle is a hydrophobic, hydrophilic or amphiphilic organic molecule.

18. Composition according to any one of claims 13 to 17, **characterized in that** it is a colloidal suspension of nanoparticles and/or microparticles and/or micelles of the polyamino acid(s) in an aqueous phase.

19. Composition according to any one of claims 13 to 18, **characterized in that** it is in the form of a solution, a gel, a suspension, an emulsion, micelles, nanoparticles, microparticles, an implant, a powder or a film.

20. Composition according to any one of claims 13 to 19, **characterized in that** it is in a form adapted to administration by the oral, parenteral, nasal, vaginal, ocular, subcutaneous, intravenous, intramuscular, intradermal, intraperitoneal, intracerebral or buccal route.

21. Composition according to any one of claims 13 to 20, **characterized in that** it is in the form of a solution in a biocompatible solvent and **in that** it is in a form adapted to injection by the subcutaneous or intramuscular route or into a tumor.

22. Composition according to any one of claims 13 to 21, **characterized in that** it is injectable and **in that** it is capable of forming a depot at the injection site.

23. Composition according to any one of claims 13 to 22, **characterized in that** it is intended for the preparation of:
• drugs, particularly for administration by the oral, nasal, vaginal, ocular, subcutaneous, intravenous, intramuscular, intradermal, intraperitoneal or intracerebral route, it being possible in particular for the active principles of these drugs to be proteins, glycoproteins, proteins bonded to one or more polyalkylene glycol chains {e.g. polyethylene glycol (PEG)}, peptides, polysaccharides, liposaccharides, oligonucleotides, polynucleotides and hydrophobic, hydrophilic or amphiphilic non-proteinaceous organic molecules;
• and/or nutriments;
• and/or cosmetic or phytosanitary products.

24. Process for the preparation of:
• drugs, particularly for administration by the oral, nasal, vaginal, ocular, subcutaneous, intravenous, intramuscular, intradermal, intraperitoneal or intracerebral route, it being possible in particular for the active principles of these drugs to be proteins, glycoproteins, proteins bonded to one or more polyalkylene glycol chains {e.g. polyethylene glycol (PEG) chains}, peptides, polysaccharides, liposaccharides, oligonucleotides, polynucleotides and hydrophobic, hydrophilic or amphiphilic non-proteinaceous organic molecules;
• and/or nutriments;
• and/or cosmetic or phytosanitary products,
**characterized in that** it consists essentially in using at least one polyamino acid according to any one of claims 1 to 12 and/or the composition according to any one of claims 13 to 22.

## Patentansprüche

1. Polyaminosäure mit Asparaginsäure- und/oder Glutaminsäure-Einheiten der folgenden Formel (I): 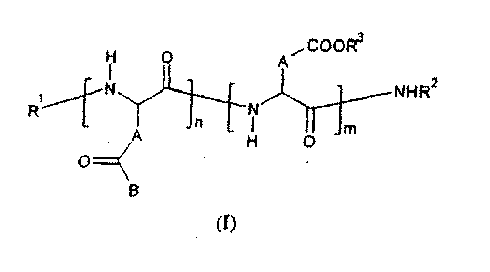 worin:
■ R¹ für H, eine lineare C₂- bis C₁₀- oder verzweigte C₃- bis C₁₀-Acylgruppe oder ein Pyroglutamat steht;
■ R² für H, lineares C₂- bis C₁₀- oder verzweigtes C₃- bis C₁₀-Alkyl, Benzyl oder eine endständige "Aminosäure"-Einheit steht;
■ R³ für H oder eine kationische Einheit steht,
■ die n Gruppen B jeweils unabhängig voneinander für einen einwertigen Rest der folgenden Formel: 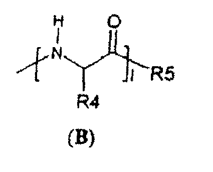 stehen, worin:
- R4 für Methyl, Isopropyl, Isobutyl, Secbutyl oder Benzyl steht;
- R5 für eine hydrophobe Gruppe mit 6 bis 30 Kohlenstoffatomen steht;
- 1 von 1 bis 6 variiert;
■ A unabhängig für -CH₂- oder -CH₂-CH₂- steht;
■ n/(n+m) als molare Pfropfrate definiert ist und von 0,5 bis 100 Mol-% variiert;
■ n + m von 3 bis 1000, vorzugsweise zwischen 30 und 300 variiert.

2. Polyaminosäure nach Anspruch 1, **dadurch gekennzeichnet, daß** in R³ die kationische Einheit unter
- Metallkationen,
- organischen Kationen
- und kationischen Polyaminosäuren ausgewählt ist.

3. Polyaminosäure nach Anspruch 1 oder 2, **dadurch gekennzeichnet, daß** in R³ die kationische Einheit unter
- Metallkationen, die unter Natrium, Kalium, Calcium und Magnesium ausgewählt sind,
- organischen Kationen, die unter
• Kationen auf Aminbasis,
• Kationen auf Oligoaminbasis,
• Kationen auf Polyaminbasis,
• Kationen auf Aminosäurebasis ausgewählt sind,
- und kationischen Polyaminosäuren, die unter Polylysin und Oligolysin ausgewählt sind, ausgewählt ist.

4. Polyaminosäure nach Anspruch 1, **dadurch gekennzeichnet, daß** die hydrophoben Gruppen R⁵ ganz oder teilweise unabhängig unter den Resten ausgewählt sind, die
■ ein lineares oder verzweigtes Alkoxy, das 6 bis 30 Kohlenstoffatome aufweist und mindestens ein Heteroatom (vorzugsweise O und/oder N und/oder S) und/oder mindestens eine Ungesättigtheit enthalten kann,
■ ein Alkoxy, das 6 bis 30 Kohlenstoffatome enthält, einen oder mehrere anellierte Carbocylen aufweist und gegebenenfalls mindestens eine Ungesättigtheit und/oder mindestens ein Heteroatom (vorzugsweise O und/oder N und/oder S) enthält,
■ ein Alkoxyaryl oder ein Aryloxyalkyl, das 7 bis 30 Kohlenstoffatome enthält und mindestens eine Ungesättigtheit und/oder mindestens ein Heteroatom (vorzugsweise O und/oder N und/oder S) enthalten kann,
umfassen.

5. Polyaminosäure nach einem der Ansprüche 1 bis 4, **dadurch gekennzeichnet, daß** die hydrophobe Gruppe des Pfropfanteils sich von einem Alkohol-Vorläufer ableitet, der unter Octanol, Dodecanol, Tetradecanol, Hexadecanol, Octadecanol, Oleylalkohol und Cholesterin ausgewählt ist.

6. Polyaminosäure nach einem der Ansprüche 1 bis 4, **dadurch gekennzeichnet, daß** die "Aminosäure"-Einheit des Pfropfanteils unter L-Leucin, L-Valin, L-Phenylalanin und Gemischen davon ausgewählt ist.

7. Polyaminosäure nach einem der Ansprüche 1 bis 6, **dadurch gekennzeichnet, daß** ihre Hauptkette aus einem alpha-L-Glutamat- oder alpha-Glutaminsäure-Homopolymer besteht.

8. Polyaminosäure nach einem der Ansprüche 1 bis 6, **dadurch gekennzeichnet, daß** ihre Hauptkette aus einem alpha-L-Aspartat- oder alpha-L-Asparaginsäure-Homopolymer besteht.

9. Polyaminosäure nach einem der Ansprüche 1 bis 6, **dadurch gekennzeichnet, daß** ihre Hauptkette aus einem alpha-L-Aspartat/alpha-L-Glutamat- oder alpha-L-Asparaginsäure-/alpha-L-Glutaminsäure-Copolymer besteht.

10. Polyaminosäure nach einem der Ansprüche 1 bis 9, **dadurch gekennzeichnet, daß** die Verteilung der Pfropfanteile tragende Asparaginsäure- und/oder Glutaminsäure-Einheiten so ist, daß die so gebildeten Polymere statistisch, blockartig oder multiblockartig aufgebaut sind.

11. Polyaminosäure nach einem der Ansprüche 1 bis 10, **dadurch gekennzeichnet, daß** ihre Molmasse zwischen 2.000 und 100.000 g/mol und vorzugsweise zwischen 5.000 und 40.000 g/mol liegt.

12. Polyaminosäure nach einem der Ansprüche 1 bis 11, **dadurch gekennzeichnet, daß** ihre molare Pfropfrate zwischen 2 und 70% und vorzugsweise zwischen 5 und 40% liegt.

13. Pharmazeutische, kosmetische, diätetische oder phytosanitäre Zusammensetzung, die mindestens eine Polyaminosäure nach einem der Ansprüche 1 bis 12 umfaßt.

14. Zusammensetzung nach Anspruch 13, **dadurch gekennzeichnet, daß** sie mindestens einen Wirkstoff umfaßt.

15. Zusammensetzung nach Anspruch 14, **dadurch gekennzeichnet, daß** der Wirkstoff über eine oder mehrere Bindungen, bei denen es sich nicht um eine kovalente Bindung bzw. kovalente Bindungen handelt, mit der Polyaminosäure bzw. den Polyaminosäuren assoziiert ist.

16. Zusammensetzung nach Anspruch 14 oder 15, **dadurch gekennzeichnet, daß** es sich bei dem Wirkstoff um ein Protein, ein Glykoprotein, ein an eine oder mehrere Polyalkylenglykolketten {vorzugsweise Polyethylenglykolketten (PEG-Ketten)} gebundenes Protein, ein Polysaccharid, ein Liposaccharid, ein Oligonucleotid, ein Polynucleotid oder ein Peptid handelt.

17. Zusammensetzung nach Anspruch 14 oder 15, **dadurch gekennzeichnet, daß** es sich bei dem Wirkstoff um ein hydrophobes, hydrophiles oder amphiphiles organisches Molekül handelt.

18. Zusammensetzung nach einem der Ansprüche 13 bis 17, **dadurch gekennzeichnet, daß** es sich dabei um eine kolloidale Suspension von Nanopartikeln und/oder Mikropartikeln und/oder Micellen der Polyaminosäure bzw. der Polyaminosäuren in einer wäßrigen Phase handelt.

19. Zusammensetzung nach einem der Ansprüche 13 bis 18, **dadurch gekennzeichnet, daß** sie in Form einer Lösung, eines Gels, einer Suspension oder einer Emulsion, in Form von Micellen, Nanopartikeln oder Mirkopartikeln, in Form eines Implantats, eines Pulvers oder eines Films vorliegt.

20. Zusammensetzung nach einem der Ansprüche 13 bis 19, **dadurch gekennzeichnet, daß** sie in einer für die Verabreichung auf oralem, parenteralem, nasalem, vaginalem, okulärem, subkutanem, intravenösem, intramuskulärem, intradermalem, intraperitonealem, intrazerebralem oder bukkalem Weg geeigneten Form vorliegt.

21. Zusammensetzung nach einem der Ansprüche 13 bis 20, **dadurch gekennzeichnet, daß** sie in Form einer Lösung in einem biokompatiblen Lösungsmittel und
in einer für die Injektion auf subkutanem oder intramuskulärem Wege oder in einen Tumor geeigneten Form vorliegt.

22. Zusammensetzung nach einem der Ansprüche 13 bis 21, **dadurch gekennzeichnet, daß** sie injizierbar ist und am Injketionsort ein Depot bilden kann.

23. Zusammensetzung nach einem der Ansprüche 13 bis 22, **dadurch gekennzeichnet, daß** sie zur Herstellung von:
• Arzneimitteln, insbesondere zur oralen, nasalen, vaginalen, okulären, subkutanen, intravenösen, intramuskulären, intradermalen, intraperitonealen oder intrazerebralen Verabreichung, wobei es sich bei den Wirkstoffen dieser Arzneimittel insbesondere um Proteine, Glykoproteine, an eine oder mehrere Polyalkylenglykolketten {vorzugsweise Polyethylenglykolketten (PEG-Ketten)} gebundene Proteine, Peptide, Polysaccharide, Liposaccharide, Oligonucleotide, Polynucleotide und hydrophobe, hydrophile oder amphiphile organische Nichtproteinmoleküle handeln kann;
• und/oder Nährstoffen
• und/oder kosmetischen oder phytosanitären Produkten
vorgesehen ist.

24. Verfahren zur Herstellung von:
• Arzneimitteln, insbesondere zur oralen, nasalen, vaginalen, okulären, subkutanen, intravenösen, intramuskulären, intradermalen, intraperitonealen oder intrazerebralen Verabreichung, wobei es sich bei den Wirkstoffen dieser Arzneimittel insbesondere um Proteine, Glykoproteine, an eine oder mehrere Polyalkylenglykolketten {vorzugsweise Polyethylenglykolketten (PEG-Ketten)} gebundene Proteine,
Peptide, Polysaccharide, Liposaccharide, Oligonucleotide, Polynucleotide und hydrophobe, hydrophile oder amphiphile organische Nichtproteinmoleküle handeln kann;
• und/oder Nährstoffen
• und/oder kosmetischen oder phytosanitären Produkten;
**dadurch gekennzeichnet, daß** man dabei im wesentlichen mindestens eine Polyaminosäure nach einem der Ansprüche 1 bis 12 und/oder die Zusammensetzung nach einem der Ansprüche 13 bis 22 verwendet.
